(19) Europäisches Patentamt

European Patent Office

Office européen des brevets

(11) Publication number: **0 343 807**
**A2**

## (12) EUROPEAN PATENT APPLICATION

(21) Application number: 89304609.4

(51) Int. Cl.⁴: **A61L 15/06**

(22) Date of filing: 08.05.89

(30) Priority: 27.05.88 US 199832

(43) Date of publication of application:
29.11.89 Bulletin 89/48

(84) Designated Contracting States:
AT BE CH DE ES FR GB IT LI NL SE

(71) Applicant: SMITH & NEPHEW UNITED INC.
1175 Starkey Road PO Box 1970
Largo Florida 34649-1970(US)

(72) Inventor: **Johns, Owen Louis**
546 Crystal Drive
Madeira Beach Florida(US)

(74) Representative: Cole, William Gwyn, Dr.
Smith and Nephew Research Limited Gilston
Park
Harlow Essex CM20 2RQ(GB)

(54) **Absorptive adhesive dressing with controlled hydration.**

(57) A nonocclusive dressing particularly effective in protecting a healing skin wound, constructed of a layer of biocompatible absorptive adhesive composition with an open backing and having an in vivo hydration rate of from about 15 to 60 g/m², hr. The absorptive adhesive composition preferably comprises, by weight, about 30-65 percent polyisobutylene, 10-30 percent polyvinylpyrollidone, 2-20 percent modified starch, 2-20 percent pectin, 0.1-10 percent acrylic elastomer, and 0-1.0 percent fiber, and may contain a treating agent such as a medicant. The dressing is preferably beveled and protected by a release layer havng tabs to permit its removal without hand contact with the absorptive adhesive layer, and may include an inlet for placing a treating solution such as a cell growth stimulator between the absorptive adhesive layer and the wound.

EP 0 343 807 A2

# ABSORPTIVE ADHESIVE DRESSING WITH CONTROLLED HYDRATION

### Background of the Invention

This invention concerns a wound dressing having a controlled hydration rate and biocompatibility which render it particularly effective for use with difficulty healing wounds such as chronic skin ulcers.

A dressing, to effectively assist a healing skin wound, should protect the wound from external contamination as well as allow the healing process to proceed. The dressing therefore should adhere to both dry and wet skin and should provide a moist environment with minimal bacterial activity to assure rapid cell growth.

Prior dressings employed for serious skin wounds have included the gauze bandage and the occlusive adhesive bandage such as the Convatec DuoDerm dressing. While both dressings adequately protect the wound from external contamination, neither provides the proper environment for rapid cell growth. The gauze bandage allows essentially uncontrolled dehydration of the wound area, causing the wound to dry out and kill the regenerating cells. The occlusive bandage, on the other hand, permits essentially no moisture loss from the wound area, causing fluid to collect in the wound and thereby promote excessive bacterial contamination of the wound. In addition, such prior adhesive bandages have not been biocompatible, causing further cell destruction and bacterial contamination.

A need therefore still exists for an improved wound dressing capable of promoting rapid healing of an external wound area while minimizing bacterial contamination of the area. It is therefore the primary objective of the present invention to provide such a dressing.

Previous absorptive adhesive dressing compositions include, for example, those disclosed in United States Patents 3339546, 3972328, 4258715, 4538603 and 4477325.

### Brief Description of the Invention

We have now found that when a healing skin wound is treated with a noncytotoxic adhesive dressing which provides controlled water evaporation from the wound area, the healing process is considerably improved.

The present invention therefore entails a nonocclusive dressing for protecting a healing skin wound, which comprises a layer of absorptive adhesive composition with a backing layer on face thereof, the absorptive adhesive composition being noncytotoxic and the dressing having an equilibrium in vivo hydration rate of from about 15 to 60 grams per square meter per hour ($g/m^2$, hr).

Preferably, the absorptive adhesive composition of the dressing comprises polyisobutylene, polyvinylpyrollidone, modified starch, pectin, acrylic elastomer and fibre, with the absorptive adhesive composition preferably also being nonhemolytic and nonpyrogenic and the dressing preferably having a hydration rate of from about 30 to 60 $g/m^2$, hr. The absorptive adhesive composition of such a dressing more preferably comprises, by weight, from about 50 to 60 percent polyisobutylene, from about 12 to 20 percent polyvinylpyrolidone, from about 4 to 10 percent modified starch, from about 10 to 20 percent pectin, from about 0.1 to 5 percent acrylic elastomer and from about 0.3 to 0.3 percent fibre.

The dressing preferably has a thickness of from about 10 to 100 mils, a protective release layer covering the second face of the absorptive adhesive layer, especially where the release layer is provided with removal means which permit the release layer to be removed by hand without contact of the absorptive adhesive composition by the hand, and a beveled edge. The dressing may further include means for placing a treating solution between the absorptive adhesive layer and the wound while the dressing is covering the wound, the treating solution preferably comprising such as a cell growth stimulator.

The present invention also emcompasses a noncytotoxic wound dressing composition, which comprises the following ingredients, in approximate weight percent:

2

| polyisobutylene | 30 - 65 |
| polyvinylpyrollidone | 10 - 30 |
| modified starch | 2 - 20 |
| pectin | 2 - 20 |
| acrylic elastomer | 0.1 - 10 |
| fibre | 0 - 1.0 |

as well as a wound dressing comprising a layer of such a composition, and a method of treating a wound area which comprises contacting the wound area with the composition.

Such a wound dressing composition is preferably also nonhemolytic and nonpyrogenic, and comprises from about 50 to 60 percent polyisobutylene, from about 12 to 20 percent polyvinylpyrollidone, from about 4 to 10 percent modified starch, from about 10 to 20 percent pectin, from about 0.1 to 5 percent acrylic elastomer and from about 0.3 to 0.8 percent fibre. Of particular value is a biocompatible wound dressing composition which comprises, by weight, from about 54 to 58 percent polyisobutylene, from about 14 to 18 percent polyvinylpyrollidone, from about 5 to 8 percent modified starch, from about 14 to 18 percent pectin, from about 05. to 2 percent cyanoacrylamide and from about 03. to 0.5 percent carded regenerated cellulose.

The wound dressing composition may include a treating agent selected from a medicant, such as an antibacterial agent and a cell growth stimulator. The treating agent in such a composition may conveniently be in time-release form.

In its broadest aspect, the present invention contemplates an absorbent dressing capable of adhering to skin, the dressing having an equilibrium in vivo hydration rate of from about 15 to 69 g/m$^2$, hr.

Brief Description of the Drawings

The features and advantages of the wound dressing and absorptive adhesive composition of the present invention will be more clearly understood from the following detailed description and accompanying drawings, in which:

Fig. 1 is a top plan view of a preferred embodiment of the absorptive adhesive wound dressing of the present invention with its protective release layer;

Fig. 2 is a cross-sectional view of the dressing of Fig. 1 taken along the line 2-2, with the thickness of the layers in this and Fig. 1 exaggerated for clarity;

Fig. 3 is a cross-sectional view as in Fig. 2 with the release layer removed and the dressing in place on a wound site;

Fig. 4 is a top plan view of a variant of the dressing of Fig. 1 which includes means for placing a treating solution at the wound site.

Fig. 5 is a cross-sectional view of the dressing of Fig. 4 taken along the line 5-5;

Fig. 6 is a bottom plan view of a variant of the dressing of Fig. 1 which includes removal tabs on the release layer; and

Fig. 7 is a cross-sectional view of the dressing of Fig. 6 taken along the line 7-7.

Detailed Description of the Invention

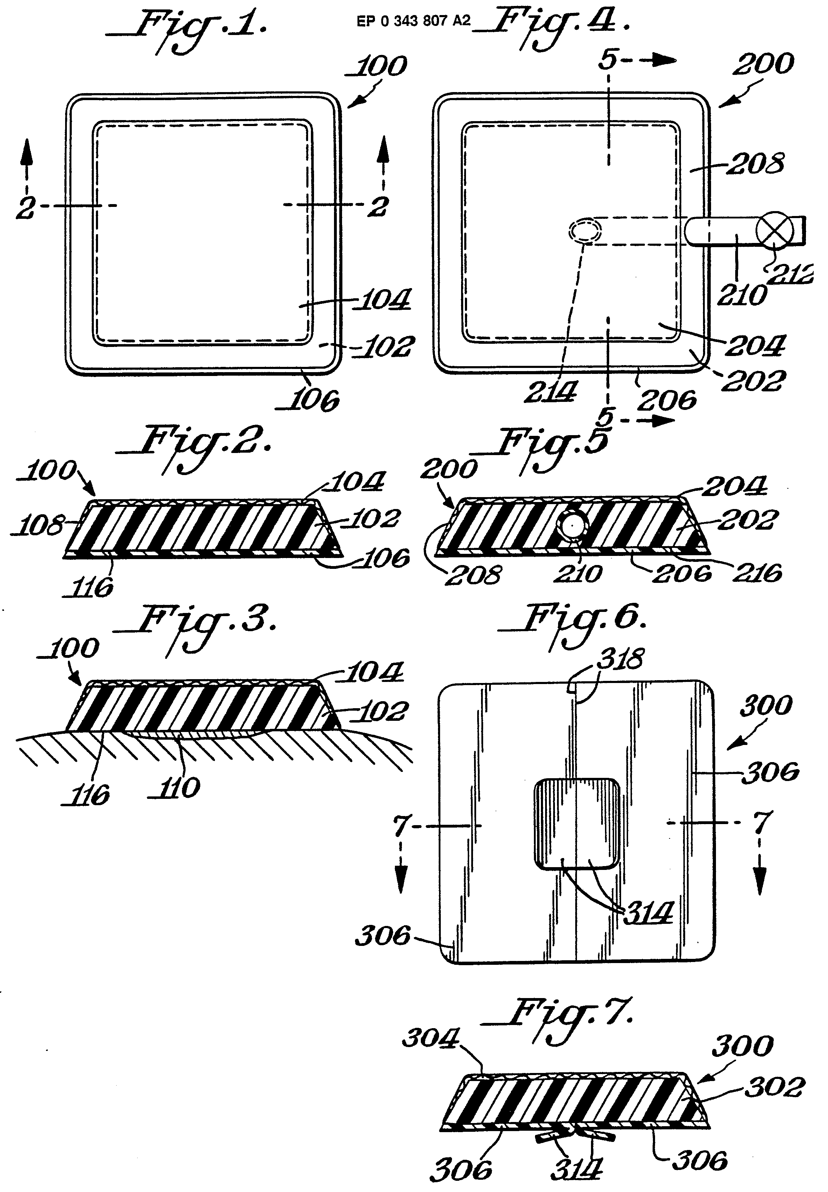

Absorptive adhesive wound dressing 100 shown in Figs. 1 and 2 comprises adhesive layer 102 of an absorptive adhesive composition with nonocclusive backing layer 104 on its one face. Release layer 106 on the other face of adhesive layer 102 helps protect adhesive layer 102 from physical damage and bacterial contamination until dressing 100 is used. Dressing 100 with its release layer 106 is normally sealed within a suitable package, not shown, then sterilized by such as chemical vapour or irradiation prior to storage.

Dressing 100, after release layer 106 is removed will normally have a thickness of from about 10 to 100 mils (254 to 2540 microns), backing layer 104 normally being from about 2 to 10 mils (51 to 254 microns), preferably about 5 mils (127 microns), and adhesive layer 102 from about 8 to 90 mils (203 to 2290 microns), preferably about 50 mils (1270 microns).

3

Bevel 108 on each of the four sides of dressing 100 helps to prevent edge damage to dressing 100, especially one of greater thickness, while dressing 100 is in place on a wound site 110, as shown in Fig. 3. this beveling is of particular benefit when dressing 100 is kept on wound site 110 for several days before being replaced. Bevel 108 is preferably obtained by a coining operation which squeezes adhesive layer 102 out from under backing layer 104 at the edges of dressing 100, allowing backing layer 104 to completely cover adhesive layer 102, even at bevel 108, and thereby prevent problems caused by exposed adhesive.

To be effective, dressing 100, ie. the combination of adhesive layer 102 and backing layer 104, must have an equilibrium in vivo hydration rate of from about 15 to 60 grams per square meter per hour ($g/m^2$, hr), preferably from about 30 to 60 $g/m^2$, hr. Hydration rates much above 60 $g/m^2$, hr cause drying of the wound area with resultant destruction of forming cells, while rates considerably below about 15 $g/m^2$, hr cause excess fluid accumulation in the wound wih unacceptable bacterial activity.

By in vivo hydration rate is meant the evaporation rate, or rate of water vapour loss, through the exposed side of backing layer 104 of dressing 100, preferably expressed in $g/m^2$, hr when the contact side of adhesive layer 102 is in contact with wound site 110, the rate being measured with the ServoMed Evaporimeter marketed by SevoMed Inc., Warrenton, VA. The equilibrium hydration rate is that essentially constant evaporation rate measured after dressing 100 has been in contact with wound site 110 for an extended period, usually from about 8 to 20 hours, and an equilibrium between moisture pickup from wound site 110 and water evaporation from backing layer 104 has been established.

In an alternative method of measurement, an in vitro equilibrium hydration rate for dressing 100 may also be determined. This determination employs the technique described above for the in vivo determination except that the free surface of adhesive layer 102 is placed in contact with the surface of a pool of distilled water at about 25°C rather than in contact with wound site 110. Testing to date indicates that the in vitro hydration rate for a given dressing 100 is about half the in vivo rate.

In addition to the required equilibrium hydration rate of from about 15 to 60 $g/m^2$, hr dressing 100 should have an adhesive layer 102 which is noncytoxic. Preferably, adhesive layer 102 is biocompatible, ie. nonhemolytic and nonpyrogenic as well as noncytotoxic. Test procedures for establishing the biocompatibility of adhesive layer 100 are indicated hereinafter.

Such controlled hydration rate and biocompatibility are readily obtained through the proper selection of the absorptive adhesive composition for adhesive layer 102 and of backing layer 104.

For adhesive layer 102 to be effective, it should possess certain properties. It should have dry tack, which is the ability to adhere to dry skin, in order to properly seal the periphery of wound site 110 against contamination from intact skin and to prevent wound exudate from seeping onto the intact skin. It should have wet tack, which is the ability to adhere to moist skin, in order to prevent dressing 100 from loosening and losing it seal during use. Is should be water absorbent in order to act as a reservoir for excess wound exudate, absorbing moisture from wound site 110 and releasing it through backing layer 104 at such a rate as to keep wound site 110 moist without fluid buildup and resultant bacteria proliferation under dressing 100. It should form a firm, solid gel with sufficient gel strength to insure mechanical integrity during the healing process and upon removal of dressing 100. It should swell uniformly in all three dimensions in order to avoid dead spaces in dressing 100 during use. And, as indicated above, it should be noncytotoxic and should preferably also be nonhemolytic and nonpyrogenic.

A suitable absorptive adhesive composition for adhesive layer 102 comprises a combination of polyisobutylene, polyvinylpyrollidone, modified starch, pectin, acrylic elastomer, and preferably fibre. This combination is particularly effective since each of the ingredients is, or can be made, biocompatible.

Polyisobutylene provides the dry tack in the preferred composition of the present dressing, polymers with molecular weights of from about $10^4$ to $10^6$ being suitable. The polyisobutylene content of the composition should be from about 30 to 65 weight percent, a content of from about 50 to 60 percent, especially from about 54 to 58 percent, being preferred. Polyisobutylene contents much below 30 percent provide too little dry tack, while contents much greater than 65 percent provide so much dry tack the dressing cannot be removed intact or without damaging the skin.

Polyvinylpryollidone (PVP) provides some wet tack and, more importantly, assures a strong gel integrity. To accomplish this, PVP contents of from about 10 to 30 weight percent should be used. With PVP contents much below 10 percent, the gel lessens its integrity. PVP contents much above 30 percent tend to dry the wound and should therefore be avoided. The preferred PVP content is from about 12 to 20 percent, especially from about 14 to 18 percent.

The modified starch in the present preferred composition is its primary water absorbent and controls the absorption rate of liquid from the wound. Particularly suitable as this absorbent modified starch are the grafted starches, or starch graft copolymers, such as saponified starch-polyacrylonitrile graft copolymers, capable of absorbing from 50 to 1,000 g fluid/g polymer. To be effective, the modified starch content of the composition should be from 2 to 20 weight percent. Contents much below 2 percent provide little water absorption, while contents much above 20 percent dry the wound. The modified starch content is preferably from about 4 to 10 percent. Especially preferred in the composition is a modified starch content of from about 5 to 18 percent.

Pectin at levels of from about 2 to 20 weight percent in the present preferred composition serves to provide additional wet tack to the composition and also assists in gel formation. Pectin levels much below 2 percent have little effect, while the dry tack of the composition is adversely affected at pectin levels much above 20 percent. Preferably, pectin levels of from about 10 to 20 percent, especially from about 14 to 18 percent, are used.

Acrylic elastomer, or elastomeric polymer of copolymer of an alkyl or alkoxyalkyl ester of acrylic or methacrylic acid, is added at levels of from about 0.1 to 10 weight percent to act as a complementary elastomeric binder for the present preferred composition. Levels of the acrylic elastomer much below 0.1 percent have little binding effect, while levels much above 10 percent cause loss of the composition's adhesion and absorption properties and should be avoided. Preferably, the acrylic elastomer content is from about 0.5 to 5, especially from about 1 to 2, percent.

The present preferred composition may include up to 1.0 weight percent fibre to enhance the mass cohesive strength of the composition. Fibre contents much greater than 1.0 percent cause the dressing to adhere to the wound site and are therefore not recommended. Preferably, the fibre content is from about 0.3 to 0.8 percent, especially from about 0.3 to 0.5 percent. While any noncytotoxic natural or synthetic fibre may be used, the fibre preferably should be also nonhemolytic and nonpyrogenic, and should be strong. Suitable fibres include such as cotton, nylon and polyester. Especially preferred is a carded 2-inch (51-mm) long, 5.5 denier ($5.5 \times 10^{-7}$ kg/meter) rayon fibre.

The preferred absorptive adhesive composition for adhesive layer 102 may also include ingredients other than those indicated hereinbefore. For example, the composition may contain a treating agent, such as a medicament or a cell growth stimulator. This treating agent may be essentially uniformly distributed throughout the composition or concentrated in adhesive layer 102 at or near surface 116 of adhesive layer 102 which contacts wound site 110, and may conveniently be in time-release form. Suitable medicants wound include, for example, antibacterial agents such as the penicillin and bacitracin antibiotics, while suitable cell growth stimulators wound include such as EGF and PDGF. Other nonessential ingredients include plasticizers such as mineral oil to facilitate processing of the adhesive composition and to increase its flexibility and toughness, and antioxidants such as butylated hydroxytoluene (BHT) to preserve the composition's shelf life.

Backing layer 104 of dressing 100 acts as a barrier to protect adhesive layer 102 against damage by the environment, such as the patient's bedding, during use. Any nonocclusive, water vapour transmitting sheet material may be used for backing layer 104, the layer preferably having a water vapour transmission rate of greater than 600 g/m², 24 hrs (90-95%·RH, 38°C). One or more layers or combinations of material may be used, backing layer 104 preferably being resistant to water and having a porous appearance. Suitable sheet materials include, for example, woven or knitted fabric of such as cotton or rayon, nonwoven fabric such as Tyvek[R], semi-reticulated or fully reticulated foam sheet, MVP film and perforated sheet such as Vispore[R].

Release layer 106 for dressing 100 may be any sheet material such as paper, polyethylene or polypropylene which will adequately protect and be properly released from adhesive layer 102. Such a suitable release material, for example, is a 40 to 75 pound basis weight paper coated on one or both sides with a suitable finish such as clay and with a release agent such as silicone. Release layer 106 will normally have a thickness of from about 2 to 6 mils (51 to 152 microns).

A variant of the nonocclusive dressing of the present invention, shown in Figs. 4 and 5, is dressing 200, with its adhesive layer 202 and backing layer 204, which includes means for placing a treating solution at a wound site between adhesive layer 202 and the wound surface (not shown). The treatment means comprises tube 210, which pierces backing layer 204 and adhesive layer 202 at bevel 208 and extends slightly downwardly through adhesive layer 202 to the centre region of dressing 200. Flow of the treating solution to dressing 200 is controlled by valve 212, the treating solution being fed to tube 210 by gravity of pump means (not shown) from a reservoir (not shown) through suitable connective tubing (not shown). The exit tip portion 214 of tube 210 is beveled such that it is essentially parallel to and slightly above surface 216 of adhesive layer 202 which contacts the wound surface. The treating solution fed through tube 210 into adhesive layer 202 may contain any agent, such as a medicament or cell growth stimulator as discussed hereinbefore, which aids in wound healing.

In its broadest sense, the nonocclusive absorptive wound dressing of the present invention need not necessarily have an absorptive adhesive layer per se. For example, the dressing might comprise a layer of polyurethane foam containing enclosed or imbedded absorbent material such as carboxymethylcellulose (CMC) or modified starch and be coated with discontinuous medical grade acrylic pressure-sensitive adhesive. Another variant might comprise a laminated structure of porous film such as Vispore alternating with layers of absorbent material such as CMC or cotton. In either case, the dressing would have a controlled in vivo hydration rate of from about 15 to 60/m², hr.

The present nonocclusive dressing may be protected by a release layer provided with a means for its removal from the dressing which permits the removal with assurance that the user's hand will not contact the absorptive adhesive composition of the dressing. An example of such a protected dressing is shown in Figs. 6 and 7, in which dressing 300, comprising adhesive layer 302 and its backing layer 304, is protected by release layer 306. In this case, release layer 306 comprises two essentially equal sections, each section of release layer 306 being provided with a pull tab 314 centrally attached to inside edge 318. Pull tab may be of the same material and integral with release layer 306, or may be of another suitable material and attached to release layer 306 in any suitably manner, such as with pressure-sensitive adhesive. Similarly, pull tab 314 may extend the full length of inside edge 318 or take another form such as a string or strip.

The following examples are merely illustrative and are not to be contrued as limiting the present invention, the scope of which is defined in the appended claims.

Example 1

An absorptive adhesive material of the following composition was prepared, blended an extruded in a clean environment using aseptic technique.

| Ingredient | Wt % |
|---|---|
| polyisobutylene | |
| A, MW 50,400–55,800 [1] | 50.9 |
| B, MW 750,000–1,050,000 [2] | 5.5 |
| polyvinylpyrollidone (PVP) [3] | 16.0 |
| pectin [4] | 16.0 |
| modified starch [5] | 6.0 |
| acrylic elastomer [6] | 1.5 |
| regenerated cellulose fibre [7] | 0.4 |
| mineral oil [8] | 3.0 |
| antioxidant [9] | 0.7 |

In this preparation, 5.0 lb (2.27kg) of polyisobutylene A was blended with 5.5 lb (2.50kg) polyisobutylene B on a suitable milling apparatus, while 1.5 lb (0.68kg) polyisobutylene A was similarly blended with 1.5 lb (0.68kg) acrylic elastomer. In a separate operation, 0.4 lb (0.18kg) regenerated cellulose fibre was carded.

Some 4.4 lb (2.00kg) polyisobutylene A was added to a kneader type mixer and mixed for 2 minutes. The 10.5 lb (4.77kg) of blended polyisobutylene A/B was then added, and the mixture was mixed 3 minutes. Subsequent additions, with blending times, included 16.0 lb (7.26kg) pectin slowly, for 5 minutes; 6.0 lb (2.72 kg) modified starch, for 5 minutes; 16.0 lb polyvinylpyrollidone slowly, for a total of 20 minutes; 0.4 lb (0.18kg) carded regenerated cellulose fibres very slowly, tearing the cellulose into pieces as it was added, for a total of 10 minutes; 3.0 lb (1.36kg) mineral oil slowly for 10 minutes; 0.7 lb (0.32kg) antioxidant, for 5 minutes and finally the 3.0 lb (1.36kg) of blended polyiosbutylene A/acrylic elastomer in small pieces, for 15 minutes until a homogeneous blend was achieved.

The blended adhesive composition was removed from the mixer while still hot and malleable from the mixing, formed into logs about 12 inches (30.5cm) long and 1.5 inches (4.1cm) thick on the release side of silicon release paper [10], and stored; the logs were then extruded onto the release paper to a thickness of 50 mils (635 microns) and a width of 4 inches (10.2cm). The extrudate was laminated with nonocclusive cloth fabric [11], coined to produce beveled edges, and finally cut to size within the registered coin area to produce individual dressings 4 in x 4 in (10cm x10cm) as shown in Figs. 1 and 2.

(1) VISTANEX[R] LMMH-LC, molecular weight 50,400-55,800 (Flory), Exxon Chemicals Americas, Houston, TX

(2) VISTANEX[R] L-80, molecular weight 750,000-1,050,000 (Flory), Exxon Chemicals Americas

(3) PLASDONE K-90 (Povidone USP), GAF Corporation, Wayne, NJ

(4) USP, Hercules, Wilmington, DE

(5) WATER LOCK[R] D-242, Grain Processing Corporation, Muscatine, IA

(6) CYANACRYL[R] C, American Cyanamid Company, Wayne, NJ

(7) Code 2664, Avtex Fibers, Inc., Charlotte, NC

(8) White USP, Witco Manufacturing, Sonneborn DIV., Petrolia, PA

(9) CAO-3 BHT, Sherwin Williams Chemicals, Fords, NJ

(10) Polyslick Release Paper, James River Corporation, H.P. Smith DIV., Bedford Park, IL

(11) Cloth-Style 3770, Adele Knits, Winston-Salem, NC

Example 2

The dressing prepared in Example 1 was compared with prior wound dressings for gel properties, biocompatibility and hydration rate, with the following results:

|  | Ex 1 | Prior 1 [1] |
|---|---|---|
| Gel Strength [2] | | |
| Gel integrity | strong | weak |
| Water clarity | good | poor |
| Gel uniformity | yes | yes |
| Absorption | good | good |
| Structural integrity [3] | | |
| % loss | 5-10 | 30-70 |

|  | Ex 1 | Prior 1 [1] |
|---|---|---|
| Absorption rate, $g/m^2$,hr [4] | | |
| 1 hr | 1140 | 1450 |
| 2 hr | 1080 | 780 |
| 3 hr | 1070 | 360 |
| Biocompatibility | | |
| Cytotoxicity [5] | nontoxic | toxic |
| 24 hr | -- | ± |
| 48 hr | -- | + |
| 72 hr | -- | + |

8

Hemolysis [6]

Pyrogenicity [7]

   maximun rise

     3 hr, °C                 0.5        1.0

     total rise, °C        0.8        2.6

Hydration rate (in vitro) [8], [9]

|  | | | | | MVT |
| --- | --- | --- | --- | --- | --- |
| $g/m^2$, hr at | Ex 1 | Prior 1 | Prior 2 | Prior 3 | Film |
| 3 hr | 2 | 1 | 3 | 0 | 2 |
| 7 hr | 21 | 2 | 5 | 0 | 3 |
| 9 hr | 26 | 3 | 6 | 0 | 4 |
| 13 hr | 26 | 3 | 6 | 0 | 3 |
| 24 hr | 26 | 3 | 6 | 0 | 4 |
| 48 hr | 27 | 3 | 6 | 0 | 3 |
| 72 hr | 27 | 3 | 6 | 0 | 3 |

(1) DuoDERM [R] hydroactive [TM] dressing, Convatec [TM] Cat. No. 187610, E.R. Squibb and Sons, Inc., Princeton, NJ

(2) Gel Strength: The test material is adhered onto the top of a water-filled, wide-mouthed glass container allowing the exposed adhesive to directly contact the distilled water for 1-4 days at room temperature. Visual observations are made noting gel intergity,

clarity of water (particulates), gel uniformity and absorption capability.

(3) <u>Structural Integrity (accelerated erosion)</u>: A pre-weighed 1 inch (2.54-cm) square test sample is placed in a beaker of distilled water and stirred on a magnetic stir plate for 3 hours. The test sample is removed and the liquid filtered. Gravimetric measurement is used to calculate the amount of eroded material, reported as percent loss.

(4) <u>Absorption Rate (liquid uptake of the matrix)</u>: A pre-weighed 1-inch (2.54cm) square prepared test sample is placed in undisturbed distilled water at room temperature. At 1, 2 and 3 hours, gravimetric measurements are recorded of the test sample and the rate reported as $g/m^2$, hr.

(5) <u>Cytotoxicity (MEM Elution - MT023; North American Science Association, Inc. [NAmSA], Northwood, OH)</u>: A 37°C extract of the test sample in Minimum Essential Medium and bovine serum is prepared. A monolayer of MRC-5 Human Embryonic Lung Cells is exposed to the centrifuged extract and examined microscopically for cytotoxic effect at 24, 48 and 72 hours.

(6) <u>Hemolysis (in vitro Direct Contact Method;</u>

NAmSA): A 0.5 g test sample is place in tubes containing 10 ml Sodium Chloride Injection. to each tube is added 0.2 ml of rabbit blood. The tubes are gently mixed by inversion and placed at 37°C for 1 hour. The blood saline mixture is then centrifuged for 5 minutes at 2,200 RPM. The absorbance of each sample solution is measured spectrophotomatrically at 545 nm.

(7) Pyrogenicity (Test T10, Current USP;NAmSA): An extract of the test sample is prepared in sterile nonpyrogenic saline. An appropriate dose (10 ml/kg) adjusted to 37°C is injected intravenously into 3 rabbits. Rectal temperatures are recorded electronically prior to injection and hourly for 3 hours. The extract is pyrogenic if any rabbit shows a 0.6°C or higher rise in temperature of if the total temperature rise of all 3 rabbits is in excess of 1.4°C.

(8) Hydration Rate (in vitro): This is measured using a ServoMed Evaporimeter (ServoMed, Inc., Warrenton, VA), which is designed to measure directly the evaporation of water from the surface of an object. A 2-inch (5.1-cm) square sample is cut from each dressing and placed over a circular cup

having a diameter of 0.75 in (1.61 cm). The cup, being completely filled with 0.9% saline, maintains fluid contact with the adhesive surface of the sample. The dressing forms a tight seal with the rim of the cup. Each test system is fitted with an input tube to maintain maximum fluid level. Hydration measurements are taken directly above the cup with an evaporimeter at 3, 7, 9, 13, 24, 48 and 72 hours.

(9) Ex 1 - Dressing prepared as in Example 1

Prior 1 - DuoDERM occlusive dressing; Convatec Cat. No. 187610

Prior 2 - Restore $^{TM}$ occlusive wound care dressing; Cat. No. 9930; Hollister Inc., Libertyville, IL

Prior 3 - Intact $^{TM}$ occlusive wound dressing; Cat. No. 740054; C.R. Bard Inc, Murray Hill, NJ

MVT film - Uniflex $^{TM}$; Cat. No. 5620; United Division of Hospital Products Group, Pfizer Inc., Largo, FL

The dressing prepared in Example 1 was compared with prior wound dressings for in vivo hydration rate and bacterial proliferation, the following results clearly showing the superiority of the dressing of the present invention.

In this comparison, dorsal wounds were surgically made on Sprague Dawley rats by excising a 1 in x 1 in (2.54cm x 2.54cm) test site using a Brown dermatome set to a depth of 0.015 in (0.4mm). After achieving hemostasis, the wounds were contaminated using bacterial challenges of $10^{1.65}$ to $10^{3.3}$ of Staphlococcus aureus. After 20 minutes following contamination, the dressings were applied over the wounds and left undisturbed for 4 days, at which time the wounds were analyzed. ServoMed Evaporimeter measurements of in vivo hydration rate were taken directly over the test dressings and over the open wound. Bacteria counts were taken for the dressing matrix, a swab of the wound, and the tissue. The recovered bacteria is reported as log 10 cfu/cm$^2$ (swab) and log 10 cfu/g (tissue).

| Dressing [1] | Hydration Rate [2] g/m2, hr | Bacteria Count [3] Matrix | Swab | Tissue |
|---|---|---|---|---|
| Open wound | 90 | NA | – | 4.1 |
| Ex 1 | 40 | 8.4 | 3.7 | 3.1 |
| Ex 1-occ | 1.5 | 6.5 | 5.5 | 5.1 |
| Prior 1-nonocc | 56[4] | 9.0 | 5.0 | 4.9 |
| Prior 2 | 1.8 | 9.2 | 6.5 | 6.0 |
| Gauze | 70 | NA | 3.5 | 4.4 |
| Gauze-occ | 0 | 4.3 | – | 4.1 |

(1) Dressing:

Ex 1 – the dressing of Example 1

Ex 1-occ – the dressing of Example 1 with an occlusive film backing

Prior 1-nonocc – DuoDERM dressing (Cat. 187610) adhesive layer with porous backing

Prior 2 – intact dressing (cat. 740054)

Gauze – nonocclusive gauze dressing; Topper[TM] Cat. No. 2436; Johnson and Johnson Products, Inc., New Brunswick, NJ

Gauze-occ – STERI-PAD[R] sterile gauze dressing 8522DJ occluded with Saran wrap; Johnson and Johnson

(2) in vivo hydration rate, ServoMed evaporimeter

(3) prolifertion from $10^{2.36}$ challenge

(4) day 2 measurement since matrix dissolved into wound at that time

Example 4

The preparation of Example 1 may be repeated using the following weight percents for the absorptive adhesive composition:

|  | a | b |
| --- | --- | --- |
| polyisobutylene | 65 | 40 |
| PVP | 30 | 10 |
| modified starch | 2 | 20 |
| pectin | 2 | 20 |
| acrylic elastomer | 0.1 | 10 |
| fibre | 0.9 | - |

resulting in noncytotoxic dressings of suitable hydration rate.

## Claims

1. A nonocclusive dressing for protecting a healing skin wound, which comprises a layer of absorptive adhesive composition with a backing layer on one face thereof, the absorptive adhesive composition being noncytotoxic and the dressing having an equilibrium in vivo hydration rate of from about 15 to 60 g/m$^2$, hr.

2. The dressing of claim 1 wherein the absorptive adhesive composition comprises polyisobutylene, polyvinylpyrollidone, modified starch, pectin, acrylic elastomer and fibre.

3. The dressing of claim 2 wherein the absorptive adhesive composition is nonhemolytic and nonpyrogenic.

4. The dressing of claim 2 wherein the hydration rate is from about 30 to 60 g/m$^2$, hr.

5. The dressing of claim 4 wherein the absorptive adhesive composition comprises, by weight, from about 50 to 60 percent polyiosbutylene, from about 12 to 20 percent polyvinylpyrollidone, from about 4 to 10 percent modified starch, from about 10 to 20 percent pectin, from about 0.1 to 5 percent acrylic elastomer and from about 0.3 to 0.8 percent fibre.

6. The dressing of claim 1 comprising means for placing a treating solution between the absorptive adhesive layer and the wound while the dressing is covering the wound.

7. The dressing of claim 6 wherein the treating solution comprises a cell growth stimulator.

8. The dressing of claim 1 wherein the protective release layer covers the second face of the absorptive adhesive layer.

9. The dressing of claim 8 wherein the release layer is provided with removal means which permit the release layer to be removed by hand without contact of the absorptive adhesive composition by the hand.

10. The dressing of claim 1 having a thickness of from about 10 to 100 mils.

11. The dressing of claim 1 wherein the dressing has a beveled edge.

12. A noncytotoxic wound dressing composition, which comprises the following ingredients, in approximate weight percent:

| polyisobutylene | 30 - 65 |
| --- | --- |
| polyvinylpyrollidone | 10 - 30 |
| modified starch | 2 - 20 |
| pectin | 2 - 20 |
| acrylic elastomer | 0.1 - 10 |
| fibre | 0 - 1.0 |

13. The composition of claim 12 wherein the composition is nonhemolytic and nonpyrogenic.

14. The composition of claim 12 comprising from about 50 to 60 percent polyisobutylene, from about 12 to 20 percent polyvinylpyrollidone, from about 14 to 10 percent modified starch, from about 10 to 20 percent pectin, from about 0.1 to 5 percent acrylic elastomer and from about 0.3 to 0.8 percent fibre.

15. The composition of claim 12 including a treating agent selected from a medicant and a cell growth stimulator.

16. The composition of claim 15 wherein the medicant is an antibacterial agent.

17. The composition of claim 15 wherein the agent is in time-release form.

14

18. A wound dressing, which comprises a layer of the composition of claim 12.

19. A biocompatible wound dressing composition, which comprises, by weight, from about 54 to 58 percent polyisobutylene, from about 14 to 18 percent polyvinylpyrollidone, from about 5 to 8 percent modified starch, from about 14 to 18 percent pectin, from about 0.5 to 2 percent cyanoacrylamide and from about 0.3 to 0.5 percent carded regenerated cellulose.

20. A method of treating a wound area, which comprises contacting the wound area with the composition of claim 12.

21. An absorbent wound dressing capable of adhering to skin and having an in vivo hydration rate of from about 15 to 60 g/m$^2$, hr.

EP 0 343 807 A2

Fig.1.

Fig.4.

Fig.2.

Fig.5.

Fig.3.

Fig.6.

Fig.7.